# FASCICULE DE BREVET EUROPEEN

(11) **EP 1 284 788 B1**
(45) Date de publication et mention de la délivrance du brevet: **27.08.2014**
(21) Numéro de dépôt: 01934083.5
(22) Date de dépôt: 10.05.2001
(51) Int. Cl.: A61N 1/39

(54) **TRAIN D'IMPULSIONS ELEMENTAIRES MODULEES DE DEFIBRILLATION**
FOLGE VON MODULIERTEN GRUNDIMPULSEN ZUR ENTFLIMMERUNG
MODULATED DEFIBRILLATION ELEMENTARY PULSE TRAIN

(30) Priorité: 10.05.2000 FR 0005924
(43) Date de publication de la demande: 26.02.2003
(73) Titulaire: Schiller Medical, 67160 Wissembourg (FR)
(72) Inventeur: CANSELL, Albert, F-67160 Wissembourg (FR); DASKALOV, Ivan, 1408 Sofia (BG)
(74) Mandataire: Metz, Paul
(86) Numéro de dépôt international: PCT/FR2001/001419
(87) Numéro de publication internationale: WO 2001/085251

(56) Documents cités:
- EP-A- 0 546 666
- EP-A- 0 813 892
- EP-A- 1 023 920

## Description

La présente invention se situe dans le domaine de la défibrillation cardiaque réalisée par au moins une onde d'énergie électrique provenant de la décharge d'au moins un condensateur.

On vise aussi bien la défibrillation interne qu'externe notamment transthoracique, ainsi que celle procurée par un défibrillateur implanté.

Elle concerne plus particulièrement de nouvelles formes d'ondes physiologiquement acceptables ou physiologiquement idéales obtenues à partir de la décharge d'au moins un condensateur aussi bien en régime monophasique qu'en régime biphasique.

La présente invention vise plus particulièrement mais non exclusivement les ondes de défibrillation dites biphasiques ayant selon des formes physiologiquement acceptables ou idéales. Le régime monophasique n'est cependant pas exclu.

Les formes des ondes de défibrillation visées sont pour la plupart connues dans leur allure générale. Elles peuvent être perfectionnées dans le détail de leur tracé pour devenir des courbes adaptées, optimales ou idéales pour une seule ou pour toute une catégorie d'applications. La ou les formes retenues pour les ondes de défibrillation sont telles que leurs performances procurent une efficacité de défibrillation élevée.

Il a été décrit dans une demande récente du déposant des impulsions ou des séries d'impulsions de défibrillation constituées par une onde de type biphasique dont les deux phases de polarités opposées sont découpées ou hachées à une fréquence élevée.

Ce dernier type d'onde, bien que donnant de bons résultats de défibrillation, est susceptible d'être amélioré encore davantage, grâce à la présente invention.

En effet, une contrainte imposée à l'onde biphasique découpée telle qu'indiqué ci-dessus, provient du fait que l'onde de défibrillation est une décharge de condensateur à travers une résistance, celle constituée par le patient. Il faut savoir que l'utilisation d'une décharge de condensateur est incontournable pour générer une telle onde au moyen d'un défibrillateur portable de faible encombrement fonctionnant sur batterie, car seul l'effet de stockage d'énergie dans un temps relativement long (par exemple 10s) et la restitution dans un temps court (quelques millisecondes) permet d'atteindre les niveaux de puissance instantanée pouvant aller jusqu'à environ 100 kW, tels qu'ils sont requis pour parvenir à une défibrillation efficace.

Cependant, cette forme d'onde à décroissance de type exponentielle, tronquée après une durée déterminée, constituant l'onde de défibrillation n'est pas optimale sur un plan physiologique. En effet, le début de cette onde présente un niveau bien plus élevé que sa fin. Ce qui veut dire que le début correspond certainement à une valeur de tension, de courant ou d'énergie qui dépasse largement le seuil nécessaire, alors que la fin de cette onde présente probablement une valeur située en dessous du seuil requis.

En d'autres termes, la " force ", terme utilisé pour désigner l'amplitude des diverses grandeurs électriques mises en jeu par la défibrillation, sera excessive au début de la 1^{ère} phase et, selon les cas, éventuellement même nocive, alors que la " force " de la défibrillation à la fin de cette phase sera insuffisante.

Le but général de l'invention est de pallier ces inconvénients en proposant de nouvelles formes d'impulsions permettant d'agir sur la "force" de la défibrillation tout au long de l'onde, indépendamment de la décharge exponentielle qui est à l'origine. On pourra ainsi, par exemple, égaliser la "force" de la défibrillation entre le début et la fin de l'exponentielle tronquée ou obtenir, d'une façon plus générale, une nouvelle forme de courbe prédéterminée de cette "force" de défibrillation, toujours différente de l'exponentielle tronquée qui est à l'origine et qui soit physiologiquement la plus acceptable ou optimale possible.

Pour décrire comment, dans le cadre de l'invention, on agit sur la force de la défibrillation, il faut d'abord énoncer les différentes grandeurs électriques qui sont déterminantes pour la défibrillation. Ces grandeurs sont d'abord la tension appliquée au patient et le courant qui traverse le patient. Le produit de ces deux paramètres correspond à une puissance instantanée à un endroit donné de la courbe. Si l'onde de défibrillation est continue, on peut intégrer cette puissance instantanée sur toute la durée de l'onde et on obtient alors l'énergie totale délivrée au patient. On peut aussi, si par exemple la courbe est discontinue, calculer l'énergie délivrée au patient sur un intervalle de temps de la courbe, en intégrant la courbe de puissance instantanée sur cet intervalle de temps.

Si l'on cherche à présent à créer une onde de défibrillation biphasique optimale c'est-à-dire d'efficacité maximale et de nocivité minimale, il faut d'abord respecter la règle de base selon laquelle l'énergie totale délivrée au patient est appliquée dans un temps optimal, aussi bien pour la 1^{ère} phase que pour la 2^{ème} phase. Ce temps qui dépend de divers paramètres physiologiques de la cellule, doit être de l'ordre d'environ 3 à 5 ms par exemple, pour chacune des phases (voir par exemple : A. Cansell, La Revue des SAMU ; 1997-5, 229 à 237). Pour qu'une énergie donnée puisse être délivrée dans cette durée, il faut que les amplitudes de tension et de courant, et donc de puissance instantanée tout au long de l'impulsion, soient adéquates. Ces conditions sont supposées être remplies dans la suite de l'exposé de l'invention, pour permettre de raisonner en terme d'énergie et d'appliquer, par souci de simplicité et de clarté, l'objet de l'invention à des doses d'énergie, soit élémentaires soit globales, étant entendu que les mêmes principes inventifs peuvent s'appliquer d'une façon similaire également aux autres paramètres de la défibrillation (tension, courant et puissance instantanée).

Le principe général de l'invention consiste à créer un train d'impulsions provenant du découpage d'au moins une décharge d'au moins un condensateur en vue d'une action de défibrillation à travers le coeur d'un être vivant par exemple un patient, à moduler les largeurs des impulsions élémentaires résultant du découpage et/ou les pauses consécutives à ces impulsions selon une loi prédéterminée, de manière à ce que par la juxtaposition-jonction cycle après cycle de chacun des segments représentant chacun une valeur moyenne obtenue sur chaque cycle, d'au moins une grandeur électrique déterminante pour la défibrillation on constitue dans son effet prévu sur le coeur une courbe de forme prédéterminée différente de celle d'une décharge exponentielle, cette forme étant physiologiquement adaptée pour obtenir une défibrillation efficace et une nocivité réduite.

En effet, l'application d'une modulation de durée permet d'agir sur l'effet réellement provoqué par chaque impulsion élémentaire sur les cellules myocardiques. Plus exactement, si on considère que ce qui est appelé un « cycle » élémentaire de commutation est constitué d'une impulsion élémentaire suivie de la pause consécutive, et si on prend par exemple l'énergie comme grandeur déterminante pour la défibrillation, on peut dire que le contenu d'énergie de chaque impulsion élémentaire peut être considéré physiologiquement comme réparti sur toute la durée du cycle élémentaire. Cela signifie que ce qui est appliqué au coeur est en réalité une valeur moyenne d'énergie calculée sur la durée de chaque cycle. Comme l'amplitude (tension et courant) des impulsions élémentaires est imposée par la forme de l'exponentielle, c'est donc en agissant sur la durée de chaque impulsion élémentaire et/ou sur la pause consécutive que l'on pourra doser à volonté l'énergie moyennée sur chaque cycle élémentaire. De cette manière, on pourra doser à loisir l'énergie contenue dans les cycles élémentaires de toute l'onde de défibrillation. Par conséquent, selon la loi de modulation appliquée à la décharge découpée d'un condensateur, il sera possible de tracer la courbe de l'énergie moyennée, cycle par cycle, pour la totalité de l'onde.

Ce procédé permet ainsi de former des ondes constituées d'une succession d'énergies élémentaires, correspondant à chaque cycle, dont l'amplitude varie dans le temps selon une forme de courbe prédéterminée modifiable à volonté au moyen de la loi de modulation, tout en partant d'une forme de courbe de décharge de base exponentielle imposée.

Il faut souligner que le cadre de l'invention ne s'étend pas seulement aux énergies des impulsions élémentaires, mais également à toutes les autres grandeurs électriques déterminantes pour la défibrillation : tension, courant, et puissance instantanée. Si on prend par exemple le paramètre courant, qui est fondamental en défibrillation, on peut ainsi constituer une courbe de forme prédéterminée, formée par les courants moyens élémentaires de chaque cycle de commutation, constitué d'une impulsion élémentaire suivie de la pause consécutive.

D'une manière similaire, on peut aussi prendre le paramètre puissance instantanée et constituer également une courbe de forme prédéterminée, formée par les puissances moyennes élémentaires de chaque cycle de commutation constitué d'une impulsion élémentaire suivie de la pause consécutive.

Il faut noter que les formes des courbes résultantes obtenues pour une loi de modulation donnée appliquée aux différentes grandeurs électriques déterminantes pour la défibrillation ne sont pas les mêmes. Des buts particuliers différents de résultats de défibrillation peuvent donc être recherchés selon les grandeurs choisies.

On peut noter enfin que le principe inventif objet de l'invention peut s'appliquer aussi bien à une enveloppe d'onde monophasique que biphasique.

D'autres caractéristiques et avantages de l'invention apparaîtront dans la description qui suit, donnée à titre d'exemple et accompagnée des dessins dans lesquels :
- la figure 1 est un schéma général de défibrillation à deux condensateurs selon une onde biphasique,
- la figure 2 est un graphique partiel de forme d'onde illustrant le principe du découpage variable ou celui de la modulation d'impulsion selon l'invention,
- la figure 3 est un graphe schématique explicatif du procédé de découpage à durée variable et de moyennage selon l'invention avec figuration pour trois cycles successifs des niveaux sur le cycle des valeurs moyennes de la grandeur déterminante pour la défibrillation en les matérialisant par trois segments successifs décalés en hauteur formant une courbe étagée qui sera lissée pour obtenir une courbe continue comme représenté en partie supérieure par la ligne en traits discontinus, ce lissage revenant dans les figures suivantes pour faciliter le dessin des courbes représentées,
- la figure 4 est un graphique de forme d'onde qui montre la superposition d'un exemple de modulation de la courbe de décharge d'un condensateur pour former une courbe d'énergie en forme de créneau avec représentation de la courbe de courant, la ligne horizontale en traits continus est l'énergie moyennée cycle par cycle et la ligne en traits discontinus ( ligne brisée ) est le courant moyenné cycle par cycle,
- les figures 5 et 6 sont chacune un graphique dans le cas d'une modulation identique aboutissant à la courbe selon l'invention respectivement de courant et d'énergie,
- les figures 7 et 8 sont chacune un graphique dans le cas d'une modulation identique aboutissant à la courbe selon l'invention respectivement de courant et d'énergie montrant des formes différentes,

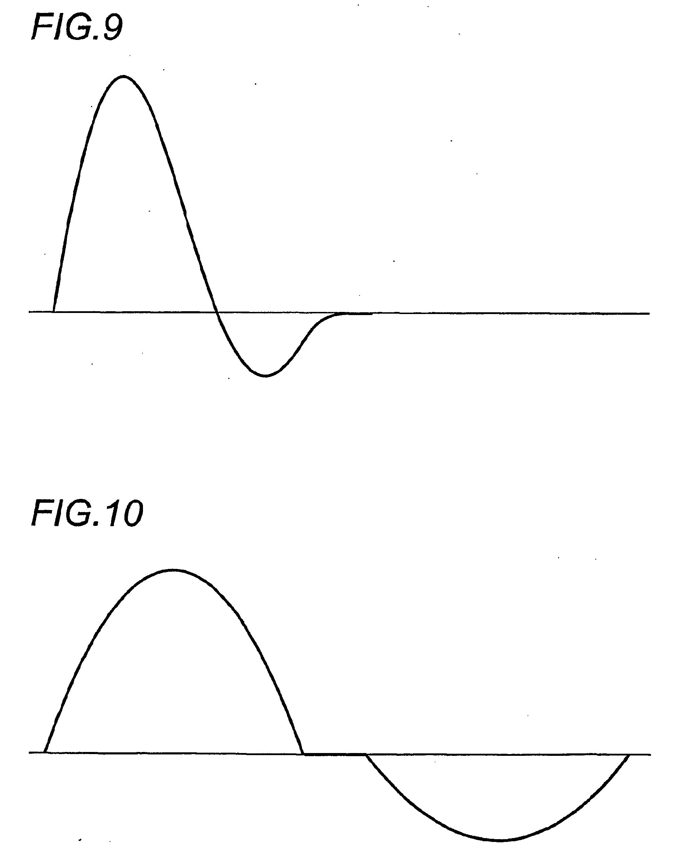

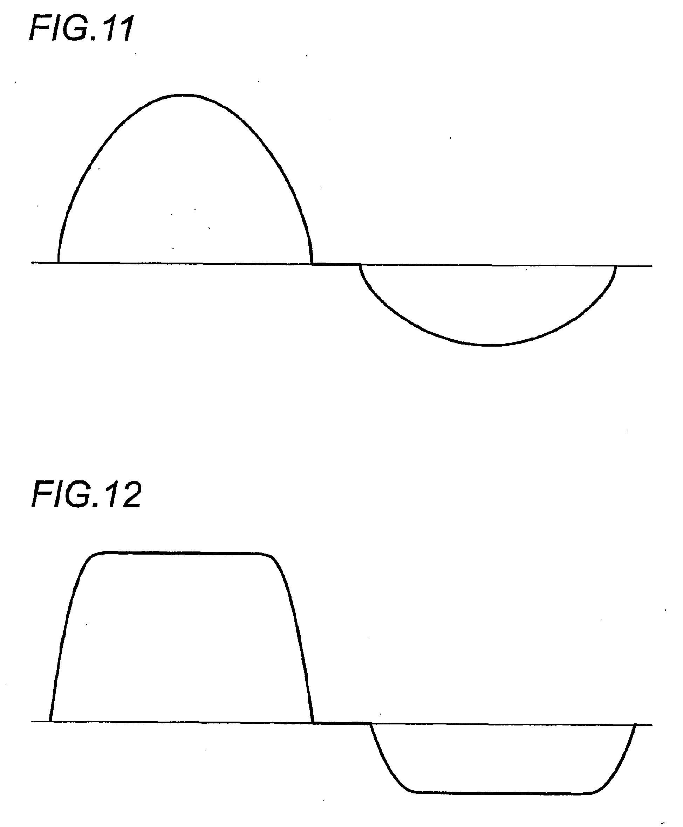

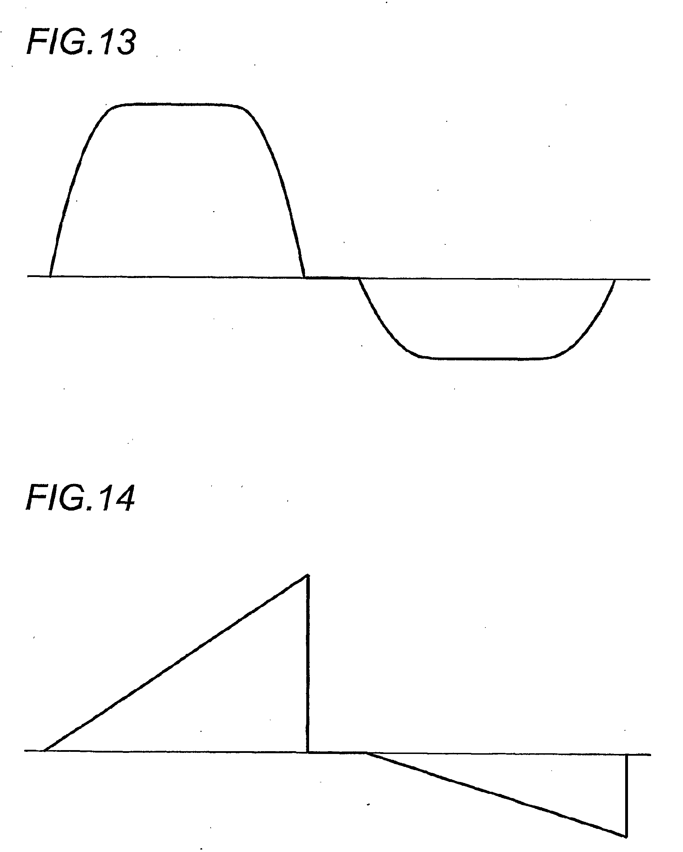

- les figures 9 à 15 sont des exemples d'allures souhaitables de courbes selon l'invention d'une des grandeurs déterminantes pour la défibrillation en fonction du temps dans une version d'onde biphasique formées chacune à partir du train d'impulsions élémentaires selon l'invention respectivement en sinusoïde sous amortie, demi-sinusoïdale, en demi-cercle, en créneau large à flancs arrondis, en créneau étroit à flancs arrondis, en rampe montante, en montée d'allure logarithmique,
- la figure 16 est un graphe illustrant le cas de formes d'onde biphasiques inversées c'est-à-dire présentant une deuxième impulsion négative se développant de façon inverse dans le temps, la forme choisie ici à titre d'exemple étant celle de la figure 15,
- la figure 17 est un graphe illustrant le cas de formes d'onde biphasiques complexes c'est-à-dire présentant une deuxième impulsion négative de forme différente choisie ici en créneau à titre d'exemple, cette courbe ayant été lissée comme les précédentes pour les besoins de la représentation graphique.

Comme déjà indiqué ci-dessus, l'invention s'étend à tous les paramètres et grandeurs électriques influant la défibrillation. En particulier les mêmes principes généraux s'appliqueront pour former à partir de la décharge du condensateur une courbe de courant, une courbe d'énergie ou de puissance instantanée de forme prédéterminée constituant l'onde de défibrillation telle que prévue dans son effet sur le coeur.

Plus particulièrement, et à titre d'exemple non limitatif dans la description ci-après, on choisit une forme de courbe concernant la variation de l'énergie dans le temps obtenue grâce à l'invention. On aurait pu aussi bien choisir une forme de courbe concernant la variation du courant dans le temps ou de la puissance instantanée. Ainsi, dès lors qu'est choisie la forme d'une courbe de variation dans le temps d'une grandeur électrique déterminante pour la défibrillation, il existe une autre courbe de variation de l'autre ou des autres grandeur(s) dans le temps.

Pour des raisons de clarté et pour bien comprendre l'invention, on définira d'abord quelques notions, termes et expressions qui seront utilisés ci-après.

Ainsi, en face de chaque expression on indiquera sa signification.

Impulsion élémentaire : il s'agit d'une impulsion d'énergie ou de courant de durée fixe ou variable suivie d'une pause également de durée fixe ou variable parmi la pluralité des impulsions élémentaires créées dont la succession forme un train d'impulsions élémentaires.

Pause : il s'agit du temps mort sans courant qui suit une impulsion élémentaire et qui sépare deux impulsions élémentaires successives. Elle est de durée fixe ou variable.

Cycle ou cycle élémentaire : il s'agit de la succession d'une impulsion élémentaire et d'une pause.

Phase : tronçon de l'onde de défibrillation dans lequel les impulsions élémentaires ont toutes une polarité donnée positive ou négative. Une onde biphasique comporte ainsi une phase positive et une phase négative.

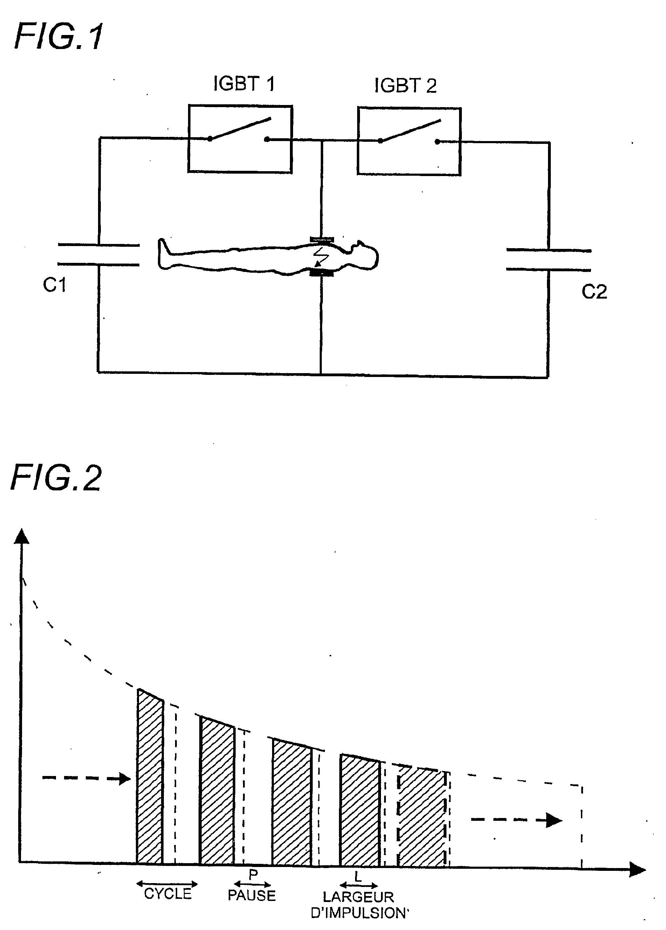

Ainsi que le circuit de la figure 1 le prévoit, l'invention se rapporte plus particulièrement, mais non exclusivement, à une onde de défibrillation dite biphasique c'est-à-dire constituée d'au moins deux phases successives de défibrillation, l'une positive et l'autre généralement de même forme mais négative et habituellement plus petite que la première.

Préférentiellement, les deux phases de défibrillation proviennent chacune de la décharge d'un condensateur. On utilise fréquemment un condensateur pour chaque polarité de phase et ceci pour des raisons de niveaux d'amplitude des deux phases de la défibrillation biphasique. Cependant l'invention n'est pas limitée à l'utilisation de deux condensateurs.

On s'intéressera ci-après à une défibrillation provoquée par deux phases successives. Il est bien entendu que l'invention couvre également une seule phase de défibrillation dite monophasique dès lors qu'elle résulte de l'application de l'invention.

L'invention procède de l'idée générale inventive qui consiste à créer au moins une phase de défibrillation et de façon préférée une onde de défibrillation monophasique ou biphasique en découpant la décharge d'au moins un condensateur pour obtenir une succession d'impulsions élémentaires d'une grandeur électrique déterminante pour la défibrillation et en modulant celles-ci et/ou les pauses consécutives selon une loi prédéfinie pour obtenir, dans son effet sur le coeur, une courbe de variation de cette grandeur électrique dans le temps qui affecte une forme prédéterminée physiologiquement acceptable, qui agit efficacement sur le coeur et présente une nocivité réduite.

Par forme prédéterminée, il faut comprendre une forme connue à l'avance parmi toutes les formes physiologiquement acceptables et surtout les formes optimales et idéales en terme de tolérance physiologique et d'efficacité de défibrillation.

On décrira maintenant l'invention dans sa généralité en référence aux figures 1, 2 et 3.

Il s'agit d'un exemple portant sur une défibrillation biphasique à partir d'ondes de défibrillation délivrées, dans le cas de la figure 1, par deux condensateurs correspondant chacun à une polarité, mais pouvant être délivrées aussi bien par un seul condensateur dont on inverse les liaisons à ses bornes.

Cette action de découpage-modulation peut être réalisée par exemple avec deux condensateurs, un pour chaque polarité c'est-à-dire pour chaque circuit de décharge contenant chacun un condensateur C1 et C2 par un commutateur électronique rapide de haute tension ou plusieurs en série, par exemple du type connu sous le sigle IGBT respectivement IGBT1 et IGBT2 piloté chacun par un circuit approprié de commande de la commutation selon une loi prédéfinie en vue d'obtenir, dans son effet sur le coeur, la forme souhaitée de la courbe résultante de variation en énergie ou en courant selon l'invention.

Il faut bien comprendre que l'on ne se limite pas à une seule grandeur électrique dont on veut imposer la forme de variation. On peut imposer la forme de variation du courant, par exemple en créneau, en cloche ou en rampe, mais on peut aussi bien choisir d'imposer la forme de variation de l'énergie élémentaire cycle par cycle, celle du courant étant alors en général d'allure différente car liée mathématiquement par une puissance d'ordre deux.

On peut imaginer que la forme souhaitée de la courbe soit modifiable pour correspondre à des cas spécifiques. Ainsi, la loi prédéfinie change pour être remplacée par une loi différente aboutissant à la formation d'une courbe de forme différente mieux adaptée à certains cas.

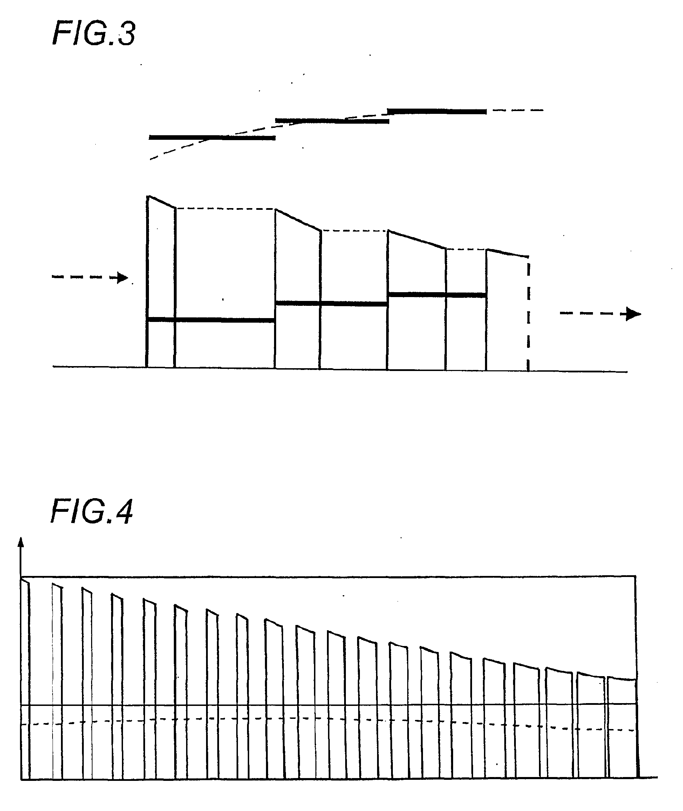

Le principe général est illustré de façon schématique par les figures 2 et 3.

Celui-ci peut à nouveau être décrit de la façon suivante. On découpe par commutation la décharge d'au moins un condensateur de façon modulée selon une loi prédéfinie. Préférentiellement, on découpe de façon variable dans le temps c'est-à-dire que l'on module les largeurs des impulsions et/ou des pauses de manière à ce que, par la juxtaposition-jonction cycle après cycle de chacun des segments représentant une valeur moyenne par moyennage sur un cycle d'au moins une grandeur électrique déterminante pour la défibrillation, on forme dans son effet prévu sur le coeur une courbe de forme prédéterminée correspondant aux meilleures conditions de défibrillation tant en efficacité qu'en tolérance physiologique.

Le processus décrit de façon théorique comme une sorte d'intégration, se produit grossièrement en pratique lors de la défibrillation par effet d'intégration dû à l'ensemble des tissus traversés jusqu'à la cellule myocardique.

Ainsi, en fonction de la forme de la courbe à obtenir, les largeurs L des impulsions élémentaires varient, ce qui est visualisé sur la figure 2 par un deuxième front descendant représenté en ligne brisée. De même, les largeurs des pauses P peuvent varier.

Le caractère hachuré des impulsions élémentaires et les deuxièmes fronts descendants en traits brisés ainsi que la représentation de la courbe de décharge sur cette figure expliquent bien visuellement le principe général inventif de l'invention.

La figure 3 a pour but de visualiser le plus simplement possible l'invention et en particulier la façon de construire la courbe de forme prédéterminée. La décharge du condensateur est découpée par commutation en impulsions élémentaires suivies chacune d'une pause. Seuls trois cycles complets ont été représentés. La variation de largeur des impulsions élémentaires a été volontairement exagérée. Les segments représentés en traits forts concernent à chaque fois le niveau de la valeur moyenne sur le cycle de la grandeur électrique choisie s'exprimant par l'impulsion élémentaire. Ces segments se succédant dans le temps forment une courbe étagée discontinue en marches d'escalier. Il s'agit de l'expression primaire de la courbe prédéterminée que l'on cherche à obtenir. Cette courbe affectera la forme prédéterminée souhaitée représentée par une ligne en traits brisés sur la figure 3.

On précise ici que la loi de modulation est telle que la profondeur de modulation est variable entre une limite basse et une limite haute, cette dernière permettant d'exploiter au maximum l'énergie disponible.

L'exemple de la figure 4 se rapporte au cas du découpage avec modulation dit découpage-modulation permettant d'aboutir à une courbe faisant partie de l'invention, ici une courbe en énergie dont la forme est en créneau rectangulaire caractérisé par une droite parallèle à l'axe des temps interrompue avec la fin tronquée de la décharge. Dans ce cas le découpage-modulation est tel que l'énergie correspondant à un cycle reste constante tout au long de l'onde c'est-à-dire que l'énergie élémentaire de chaque cycle est identique à celle du cycle précédent ou suivant. L'énergie s'établit à un niveau moyen déterminé par cette constante. Pour des raisons d'efficacité de défibrillation, ce niveau moyen sera choisi le plus élevé possible en tenant compte des paramètres particuliers du ou des circuit(s) et de la capacité du ou des condensateur(s).

Ainsi, si la hauteur de chaque impulsion élémentaire à partir d'une même décharge est importante en début de décharge, sa largeur est faible et de façon complémentaire la durée de la pause est importante. Aux environs du milieu de la courbe de décharge, le rapport entre la largeur de l'impulsion et la durée de pause est voisin de l'unité. A la fin de la durée de décharge, la durée de pause devient faible puis va même jusqu'à s'annuler. Toute la durée du cycle à ce moment est nécessaire pour que la quantité d'énergie délivrée par ce cycle soit maximale.

L'onde de défibrillation à partir de la phase de polarité opposée fournie, par exemple par le deuxième condensateur, est formée de la même façon par des impulsions élémentaires de largeur variable en augmentation jusqu'à la fin et en durée de pause dégressive pour maintenir dans chaque cycle une quantité d'énergie constante. L'énergie totale contenue dans la deuxième phase est cependant inférieure à celle de la première phase.

Sur la figure 4 a été représentée sous la forme d'une ligne en traits brisés la variation correspondante de l'autre grandeur sensible à savoir ici la variation du courant. On remarque à l'allure de la courbe que cette autre grandeur varie de façon différente en raison de la relation mathématique qui lie ces deux grandeurs.

Le principe général de l'invention s'applique de la même façon à des ondes résultantes de défibrillation selon l'invention dont les courbes d'énergie sont d'allure différente par exemple arrondies, en rampe montante... dont quelques exemples supposés intéressants ont été représentés sur les figures de 9 à 17.

Dans le cas d'une courbe de défibrillation résultante sous la forme d'une rampe montante, les impulsions élémentaires de départ seront de largeur extrêmement faible en raison de la montée progressive de la courbe à partir de zéro. L'augmentation en largeur et le resserrement progressif de celles-ci correspondent à la montée vers un maximum.

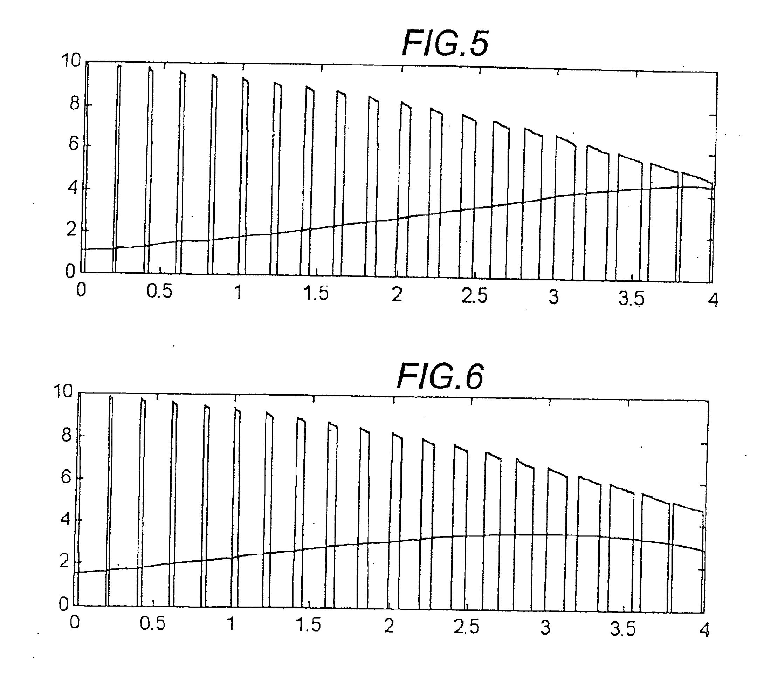

Les figures 5 et 6 puis 7 et 8 montrent la succession des impulsions élémentaires et les courbes résultantes de variation d'énergie et de courant dans le cas d'une courbe en rampe montante et dans le cas d'une courbe en montée arrondie.

Les deux exemples choisis ici portent sur des courbes présentant des variations suffisamment rapides pour être bien démonstratives visuellement du principe de découpage-modulation utilisé.

L'exemple de la rampe montante (figures 5 et 6) montre le resserrement progressif avec augmentation de largeur des impulsions élémentaires. La quantité d'énergie contenue dans chaque cycle et le courant augmentent progressivement.

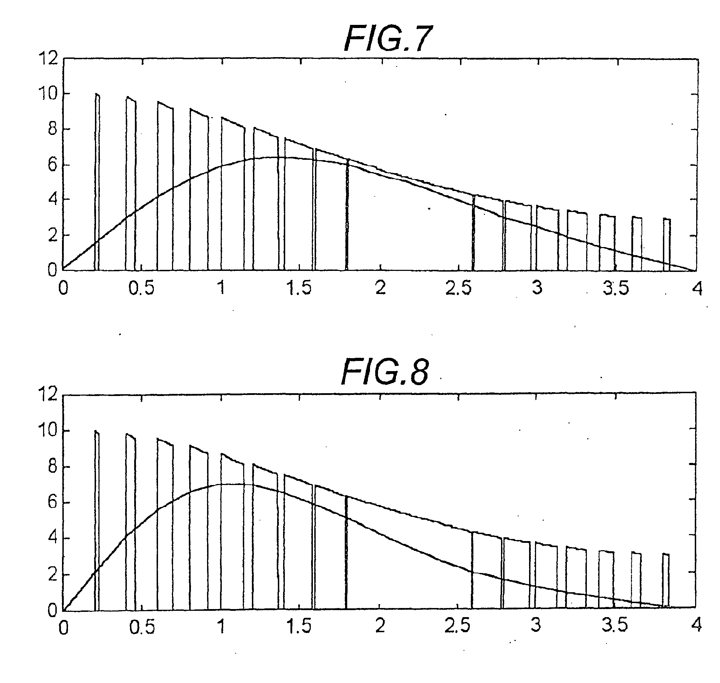

L'exemple de l'onde à dos arrondi (figures 7 et 8) montre un espacement et une finesse des premières impulsions élémentaires ainsi qu'un déserrement des impulsions élémentaires finales alors que les impulsions élémentaires se rapprochent jusqu'à se confondre en partie dans une zone proche du centre correspondant à la portion quasi linéaire descendante des courbes. L'onde résultante donne d'excellents résultats en défibrillation.

Dans ces deux cas, les courbes d'énergie et de courant se ressemblent. La transformée linéaire au carré présente la même allure que la courbe de base.

Les figures intermédiaires de 9 à 15 représentent des ondes biphasiques à titre d'exemple pouvant apporter de bons résultats dans le cadre de l'invention.

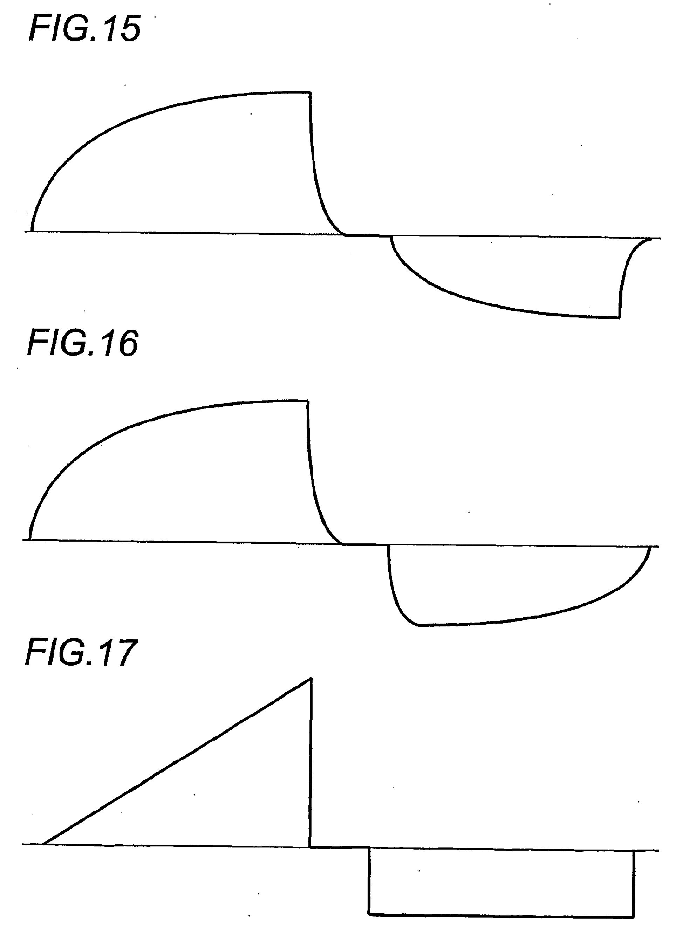

Les dernières figures 16 et 17 montrent le cas d'impulsions de défibrillation spéciales. La courbe de la figure 16 est dérivée en forme de celle de la figure 15.

Il s'agit pour la première (figure 16) de ce que l'on pourrait appeler une onde biphasique inversée c'est-à-dire dont la deuxième phase n'est pas seulement opposée en polarité mais inversée dans le sens des temps croissants.

Dans le cas présent de la figure 16, la rampe qui était montante dans la première phase devient descendante à la fin de la deuxième phase. Ce type de forme de courbe de défibrillation peut présenter une efficacité accrue.

Il s'agit ensuite, pour le cas de la figure 17, d'une courbe d'une onde biphasique complexe dans laquelle la deuxième phase peut affecter une forme quelconque. Bien entendu, on adoptera des formes adaptées c'est-à-dire celles qui produisent des améliorations dans l'efficacité de défibrillation et la baisse de nocivité pour les tissus et pour le patient.

On a représenté ici une impulsion de deuxième phase sous la forme d'un créneau négatif réputé pour son efficacité meilleure que celle procurée par une exponentielle.

L'invention se rapporte également au procédé de création de ce train d'impulsions élémentaires et de formation de la courbe de forme prédéterminée favorable à la défibrillation et moins nocive pour l'organisme et surtout pour les tissus du coeur et de la poitrine.

Le procédé se rapporte à toutes les grandeurs électriques déterminantes pour la défibrillation.

Il concerne la formation d'un train d'impulsions élémentaires de défibrillation à partir d'une décharge d'au moins un condensateur et se caractérise en ce que l'on découpe cette décharge en impulsions élémentaires séparées chacune par une pause, celles-ci étant modulées selon une loi prédéterminée, de manière à ce que la juxtaposition-jonction cycle après cycle de chacun des segments représentant chacun une valeur moyenne à chaque fois sur un cycle d'au moins une grandeur électrique déterminante pour la défibrillation constitue, dans son effet prévu sur le coeur, une courbe de forme prédéterminée différente de celle d'une décharge exponentielle, cette forme de courbe étant physiologiquement adaptée pour obtenir une défibrillation efficace et une nocivité réduite.

De façon particulière, il se caractérise en ce que les impulsions élémentaires et/ou les pauses sont modulées en durée.

En ce qui concerne les grandeurs électriques déterminantes pour la défibrillation, conformément à ce qui précède, les grandeurs retenues sont d'abord la quantité d'énergie élémentaire contenue dans chaque cycle, ensuite le courant et de façon supplémentaire la puissance instantanée des impulsions élémentaires.

Sur le schéma de la figure 1, le réservoir d'énergie pour la délivrance du choc électrique de défibrillation est représenté par deux condensateurs C₁ et C₂ de polarité opposée en raison de leur sens de montage.

L'intérêt de prendre deux condensateurs réside dans le fait que de cette manière on peut fixer librement l'amplitude de la 2^{ème} phase, et obtenir ainsi une proportion donnée de l'amplitude de cette phase par rapport à l'amplitude de la 1^{ère} phase. L'avantage résultant en sera que la 2^{éme} phase parviendra ainsi, avec cette proportion choisie optimale, à éliminer le mieux possible les charges électriques qui ont été accumulées sur la cellule cardiaque par la 1^{ère} phase, ce qui conduit à une efficacité de défibrillation maximale.

Si l'on utilise des ondes biphasiques continues usuelles (non découpées) ou si l'on utilise des ondes découpées selon un facteur de forme égal à l'unité, ce dimensionnement libre de l'amplitude de la 2^{ème} phase au moyen d'un deuxième condensateur que l'on charge à la tension voulue est le seul moyen d'obtenir une proportion donnée entre l'amplitude notamment en courant, en charge électrique ou en énergie, de la 2^{ème} phase par rapport à la 1^{ère} phase si les durées des deux phases doivent rester constantes. En effet, si l'on utilise un seul condensateur, l'amplitude de la 2^{ème} phase sera égale à l'amplitude à la fin de la 1^{ère} phase (que l'on inverse), et les proportions du courant, de la charge électrique ou de l'énergie des deux phases ne pourront pas être choisies librement.

Par contre, dans le cas du train d'impulsions modulées selon l'invention, il existe une manière d'éviter l'utilisation de deux condensateurs tout en gardant la possibilité d'obtenir une proportion prédéterminée de la 2^{ème} phase par rapport à la 1^{ère} phase, proportion exprimée soit en charges électriques, soit en énergies des deux phases.

Cette manière consiste, en utilisant un seul condensateur, à doser de façon adéquate la charge totale ou l'énergie totale nécessaire à la 2^{ème} phase en agissant sur les durées soit des impulsions individuelles de cette 2^{ème} phase, soit des pauses de la 2^{ème} phase, soit des deux à la fois, sans modifier la durée totale de cette 2^{ème} phase.

L'application de l'invention couvre extrêmement avantageusement toutes les formes cliniques de défibrillation, aussi bien ventriculaire qu'auriculaire (cardioversion), et aussi bien internes par sonde endocavitaire et/ou électrodes épicardiques et/ou électrodes sous-cutanées qu'externes par électrodes cutanées appliquées sur le thorax.

Les applications internes couvrent aussi bien celles du défibrillateur implantable que celles pratiquées au laboratoire d'électrophysiologie.

Bien entendu, l'invention englobe toutes les variantes directes et simples, la substitution par des moyens équivalents et de façon plus générale toutes modifications ou adjonctions non inventives.

## Revendications

1. Train d'impulsions élémentaires de défibrillation séparées par des pauses, train constitué de la succession de cycles impulsion-pause provenant du découpage d'au moins une décharge d'au moins un condensateur en vue d'une action de défibrillation à travers le coeur d'un être vivant par exemple d'un patient, **caractérisé en ce que** les impulsions élémentaires résultant du découpage de la décharge du ou des condensateur(s) et/ou les pauses à énergie ou potentiel nul entre ces impulsions *sont variables en* durée selon une loi *prédéterminée* de modulation, de manière à ce que par la juxtaposition-jonction, cycle après cycle, de chacun des segments de ces *impulsions* représentant chacun une valeur moyenne obtenue par moyennage sur chaque cycle, d'au moins une grandeur électrique déterminante pour la défibrillation, constituant ansi dans son effet prévu sur le coeur, une courbe de forme prédéterminée différente de celle d'une décharge exponentielle, cette forme de courbe résultante étant physiologiquement adaptée pour obtenir une défibrillation efficace et une nocivité réduite.

2. Train d'impulsions de défibrillation selon la revendication 1, **caractérisé en ce que** les impulsions élémentaires constituant le train d'impulsions sont contenues dans l'enveloppe d'une onde biphasique.

3. Train d'impulsions de défibrillation selon les revendications 1 ou 2, **caractérisé en ce que** la loi de modulation est telle que la profondeur de modulation est variable entre une limite basse et une limite haute, cette dernière permettant d'exploiter au maximum l'énergie disponible.

4. Train d'impulsions de défibrillation selon l'une quelconque des revendications précédentes **caractérisé en ce que** la courbe de forme prédéterminée est la courbe des moyennes cycle par cycle obtenues sur chaque cycle de chaque quantité d'énergie élémentaire contenue dans un cycle.

5. Train d'impulsions de défibrillation selon la revendication précédente **caractérisé en ce que** l'énergie élémentaire correspondant à un cycle est identique à celle du cycle précédent et à celle du cycle suivant.

6. Train d'impulsions de défibrillation selon l'une quelconque des revendications précédentes de 1 à 3 **caractérisé en ce que** la courbe de forme prédéterminée est la courbe des moyennes cycle par cycle obtenues sur chaque cycle de chaque valeur du courant pendant le cycle.

7. Train d'impulsions de défibrillation selon l'une quelconque des revendications précédentes de 1 à 3 **caractérisé en ce que** la courbe de forme prédéterminée est la courbe des moyennes cycle par cycle obtenues sur chaque cycle de chaque puissance instantanée du cycle.

8. Train d'impulsions de défibrillation selon l'une quelconque des revendications précédentes **caractérisé en ce que** la courbe des moyennes cycle par cycle de la grandeur électrique dans un cycle a la forme approximative d'un créneau sensiblement linéaire et parallèle à l'axe des temps.

9. Train d'impulsions de défibrillation selon l'une quelconque des revendications précédentes de 1 à 7 **caractérisé en ce que** la courbe des moyennes cycle par cycle de la grandeur électrique dans un cycle a la forme approximative d'une rampe montante.

10. Train d'impulsions de défibrillation selon l'une quelconque des revendications précédentes de 1 à 7 **caractérisé en ce que** la courbe des moyennes cycle par cycle de la grandeur électrique dans un cycle a la forme approximative d'une rampe descendante.

11. Train d'impulsions de défibrillation selon l'une quelconque des revendications précédentes de 1 à 7 **caractérisé en ce que** la courbe des moyennes cycle par cycle de la grandeur électrique dans un cycle a la forme approximative d'une demi-onde sinusoïdale.

12. Train d'impulsions de défibrillation selon l'une quelconque des revendications précédentes de 1 à 7 **caractérisé en ce que** la courbe des moyennes cycle par cycle de la grandeur électrique dans un cycle a la forme approximative d'un demi-cercle.

13. Train d'impulsions de défibrillation selon l'une quelconque des revendications précédentes de 1 à 7 **caractérisé en ce que** la courbe des moyennes cycle par cycle de la grandeur électrique dans un cycle a la forme approximative d'un dôme.

14. Train d'impulsions de défibrillation selon l'une quelconque des revendications précédentes **caractérisé en ce que** les courbes des moyennes, cycle par cycle, de la grandeur électrique obtenues sur chaque cycle dans le cas d'impulsions élémentaires contenues dans l'enveloppe d'une onde biphasique ont des formes identiques.

15. Train d'impulsions de défibrillation selon l'une quelconque des revendications précédentes de 1 à 13 **caractérisé en ce que** les courbes des moyennes cycle par cycle de la grandeur électrique obtenues sur chaque cycle dans le cas d'impulsions élémentaires contenues dans l'enveloppe d'une onde biphasique ont des formes alternées inverses ou différentes.

16. Train d'impulsions de défibrillation selon la revendication précédente **caractérisé en ce que** les courbes des moyennes cycle par cycle de la grandeur électrique obtenues sur chaque cycle dans le cas d'impulsions élémentaires contenues dans l'enveloppe d'une onde biphasique est une rampe montante correspondant à la première phase et un créneau correspondant à la deuxième phase.

17. Procédé de formation d'un train d'impulsions élémentaires de défibrillation à partir d'une décharge d'au moins un condensateur **caractérisé en ce que** l'on découpe cette décharge en impulsions élémentaires séparées chacune par une pause **à énergie ou potentiel nul**, celles-ci étant *modulées en durée selon une loi prédéterminée de modulation permettant d'exploiter au maximum l'énergie disponible*, de manière à ce que la juxtaposition-jonction, cycle après cycle, de chacun des segments de *potentiel ou d'énergie* représentant chacun une valeur moyenne obtenue à chaque fois sur chaque cycle d'au moins une grandeur électrique déterminante pour la défibrillation constitue, dans son effet prévu sur le coeur, une courbe de forme prédéterminée différente de celle d'une décharge exponentielle, cette forme de courbe résultante étant physiologiquement adaptée pour obtenir une défibrillation efficace et une nocivité réduite.

18. Procédé de formation d'un train d'impulsions élémentaires selon la revendication 17 **caractérisé en ce que** la grandeur électrique déterminante est la quantité d'énergie contenue dans chaque cycle.

19. Procédé de formation d'un train d'impulsions élémentaires selon la revendication précédente **caractérisé en ce que** l'énergie correspondant à un cycle est identique à celle du cycle précédent et à celle du cycle suivant.

20. Procédé de formation d'un train d'impulsions selon la revendication 17 ou 18 **caractérisé en ce que** la grandeur électrique déterminante est le courant de chaque cycle.

21. Procédé de formation d'un train d'impulsions selon la revendication 17 ou 18 **caractérisé en ce que** la grandeur électrique déterminante est la puissance de chaque cycle.

22. Procédé de formation d'un train d'impulsions selon l'une quelconque des revendications 17 à 21 **caractérisé en ce que**, en utilisant un seul condensateur, on agit sur les durées des impulsions individuelles de la 2^{ème} phase de manière à doser la charge électrique totale ou l'énergie totale de cette 2^{éme} phase pour que la charge ou l'énergie de la 2^{éme} phase ait une proportion voulue par rapport à la charge ou à l'énergie de la 1^{ére} phase sans modifier la durée de cette 2^{éme} phase.

23. Procédé de formation d'un train d'impulsions selon l'une quelconque des revendications de 17 à 21 **caractérisé en ce que**, en utilisant un seul condensateur, on agit sur les durées des pauses individuelles de la 2^{ème} phase de manière à doser la charge électrique totale ou l'énergie totale de cette 2^{ème} phase pour que la charge ou l'énergie de la 2^{éme} phase ait une proportion voulue par rapport à la charge ou à l'énergie de la 1^{ère} phase sans modifier la durée de cette 2^{ème} phase.

24. Procédé de formation d'un train d'impulsions selon l'une quelconque des revendications de 17 à 21 **caractérisé en ce que**, en utilisant un seul condensateur, on agit sur les durées des impulsions et des pauses individuelles de la 2^{éme} phase de manière à doser la charge électrique totale ou l'énergie totale de cette 2^{ème} phase pour que la charge ou l'énergie de la 2^{éme} phase ait une proportion voulue par rapport à la charge ou à l'énergie de la 1^{ére} phase sans modifier la durée de cette 2^{éme} phase.

## Patentansprüche

1. Folge von durch Pausen getrennten elementaren Defibrillations-Impulsen, wobei die Folge aus der Sukzession von Impuls-Pausenzyklen gebildet ist, die aus dem Zerhacken wenigstens einer Ladung aus wenigstens einem Kondensator für eine Defibrillationsaktion durch das Herz eines Lebewesens, z. B. eines Patienten, stammen, **dadurch gekennzeichnet, dass** die elementaren Impulse, die aus dem Zerhacken der Ladung des oder des (der) Kondensator(en) und / oder der Pausen mit Nullenergie oder -potenzial zwischen diesen Impulsen resultieren, *in der Dauer* gemäß einem *vorbestimmten Modulationsgesetz* derart *variabel sind,* dass jedes der Segmente durch die Aneinander-Nebeneinanderreihung Zyklus für Zyklus jedes Segments dieser *Impulse*, die jeder einen Durchschnittswert darstellen, der per Mittelbildung auf jedem Zyklus wenigstens eine für die Defibrillation ausschlaggebende elektrische Größe erhalten wird und somit in seiner auf dem Herzen vorgesehenen Wirkung eine Kurve in einer unterschiedlichen vorbestimmten Form zu der einer exponentiellen Ladung bildet, wobei diese resultierende Kurvenform physiologisch angepasst ist, um eine wirksame Defibrillation und eine reduzierte Schädlichkeit zu erreichen.

2. Folge von elementaren Defibrillations-Impulsen gemäß Anspruch 1, **dadurch gekennzeichnet, dass** die elementaren Impulse, die die Impulsfolge bilden, in der Hülle einer Zweiphasenwelle enthalten sind.

3. Folge von elementaren Defibrillations-Impulsen gemäß Anspruch 1 oder 2, **dadurch gekennzeichnet, dass** das Modulationsgesetz derart ist, dass die Modulationstiefe zwischen einer unteren Grenze und einer oberen Grenze variabel ist, wobei letztere die maximale Nutzung der verfügbaren Energie erlaubt.

4. Folge von elementaren Defibrillations-Impulsen gemäß einem der voranstehenden Ansprüche, **dadurch gekennzeichnet, dass** die Kurve in vorbestimmter Form die Kurve der Mittel Zyklus für Zyklus ist, die auf jedem Zyklus jeder elementaren Energiemenge erhalten wird, die in einem Zyklus enthalten ist.

5. Folge von Defibrillations-Impulsen gemäß dem voranstehenden Anspruch, **dadurch gekennzeichnet, dass** die einem Zyklus entsprechende elementare Energie identisch mit der des vorherigen Zyklus und der des nachfolgenden Zyklus ist.

6. Folge von Defibrillations-Impulsen gemäß einem der voranstehenden Ansprüche 1 bis 3, **dadurch gekennzeichnet, dass** die Kurve in vorbestimmter Form die Kurve der Mittel Zyklus für Zyklus ist, die auf jedem Zyklus jedes Wertes des Stroms während des Zyklus erhalten werden.

7. Folge von Defibrillations-Impulsen gemäß einem der voranstehenden Ansprüche 1 bis 3, **dadurch gekennzeichnet, dass** die Kurve in vorbestimmter Form die Kurve der Mittel Zyklus für Zyklus ist, die auf jedem Zyklus jeder Momentanleistung des Zyklus erhalten werden.

8. Folge von Defibrillations-Impulsen gemäß einem der voranstehenden Ansprüche, **dadurch gekennzeichnet, dass** die Kurve der Mittel Zyklus für Zyklus der elektrischen Größe in einem Zyklus die ungefähre Form einer deutlich linearen und zur Achse der Zeiten parallelen Lücke hat.

9. Folge von Defibrillations-Impulsen gemäß einem der voranstehenden Ansprüche 1 bis 7, **dadurch gekennzeichnet, dass** die Kurve der Mittel Zyklus für Zyklus der elektrischen Größe in einem Zyklus die ungefähre Form einer ansteigenden Rampe hat.

10. Folge von Defibrillations-Impulsen gemäß einem der voranstehenden Ansprüche 1 bis 7, **dadurch gekennzeichnet, dass** die Kurve der Mittel Zyklus für Zyklus der elektrischen Größe in einem Zyklus die ungefähre Form einer absteigenden Rampe hat.

11. Folge von Defibrillations-Impulsen gemäß einem der voranstehenden Ansprüche 1 bis 7, **dadurch gekennzeichnet, dass** die Kurve der Mittel Zyklus für Zyklus der elektrischen Größe in einem Zyklus die ungefähre Form einer sinusförmigen Halbwelle hat.

12. Folge von Defibrillations-Impulsen gemäß einem der voranstehenden Ansprüche 1 bis 7, **dadurch gekennzeichnet, dass** die Kurve der Mittel Zyklus für Zyklus der elektrischen Größe in einem Zyklus die ungefähre Form eines Halbkreises hat.

13. Folge von Defibrillations-Impulsen gemäß einem der voranstehenden Ansprüche 1 bis 7, **dadurch gekennzeichnet, dass** die Kurve der Mittel Zyklus für Zyklus der elektrischen Größe in einem Zyklus die ungefähre Form einer Kuppel hat.

14. Folge von Defibrillations-Impulsen gemäß einem der voranstehenden Ansprüche, **dadurch gekennzeichnet, dass** die Kurven der Mittel Zyklus für Zyklus der elektrischen Größen, die auf jedem Zyklus bei elementaren Impulsen erhalten werden, die in der Hülle einer Zweiphasenwelle enthalten sind, identische Formen haben.

15. Folge von Defibrillations-Impulsen gemäß einem der voranstehenden Ansprüche 1 bis 13, **dadurch gekennzeichnet, dass** die Kurven der Mittel Zyklus für Zyklus der elektrischen Größe, die auf jedem Zyklus bei elementaren Impulsen erhalten werden, die in der Hülle einer Zweiphasenwelle enthalten sind, alternierende umgekehrte oder unterschiedliche Formen haben.

16. Folge von Defibrillations-Impulsen gemäß dem voranstehenden Anspruch, **dadurch gekennzeichnet, dass** die Kurven der Mittel Zyklus für Zyklus der elektrischen Größe auf jedem Zyklus bei elementaren Impulsen erhalten werden, die in der Hülle einer Zweiphasenwelle enthalten sind, eine ansteigende Rampe ist, die der ersten Phase entspricht, und eine Lücke, die der zweiten Phase entspricht.

17. Verfahren zur Bildung einer Folge von elementaren Defibrillations-Impulsen ausgehend von einer Ladung von wenigstens einem Kondensator, **dadurch gekennzeichnet, dass** diese Ladung in elementare Impulse zerhackt wird, die jeweils durch eine Pause **mit Nullenergie oder -potenzial** getrennt werden, wobei diese *in der Dauer gemäß einem vorbestimmten Modulationsgesetz moduliest werden, das die maximale Nutzung der verfügbaren Energie derart zulässt,* dass die Aneinander-Nebeneinander-Reihung Zyklus für Zyklus jedes der Segmente mit *Potential oder Energie* maximal genutzt werden kann, die jeweils einen durchschnittlichen Wert darstellen, der jedes Mal auf jedem Zyklus wenigstens einer elektrischen Größe erhalten wird, die für die Defibrillation ausschlaggebend ist und in seiner auf dem Herzen vorgesehenen Wirkung eine vorbestimmte Form bildet, die unterschiedlich von der einer exponentiellen Ladung ist, wobei diese resultierende Kurvenform physiologisch angepasst ist, um eine wirksame Defibrillation und eine reduzierte Schädlichkeit zu erreichen.

18. Verfahren zur Bildung einer Folge von elementaren Defibrillations-Impulsen gemäß Anspruch 17, **dadurch gekennzeichnet, dass** die ausschlaggebende elektrische Größe die in jedem Zyklus enthaltene Energiemenge ist.

19. Verfahren zur Bildung einer Folge von elementaren Defibrillations-Impulsen gemäß dem voranstehenden Anspruch, **dadurch gekennzeichnet, dass** die einem Zyklus entsprechende Energie identisch mit der des vorherigen Zyklus und der des nachfolgenden Zyklus ist.

20. Verfahren zur Bildung einer Folge von Impulsen gemäß Anspruch 17 oder 18, **dadurch gekennzeichnet, dass** die ausschlaggebende elektrische Größe der Strom jedes Zyklus ist.

21. Verfahren zur Bildung einer Folge von Impulsen gemäß Anspruch 17 oder 18, **dadurch gekennzeichnet, dass** die ausschlaggebende elektrische Größe die Leistung jedes Zyklus ist.

22. Verfahren zur Bildung einer Folge von Impulsen gemäß Anspruch 17 bis 21, **dadurch gekennzeichnet, dass** unter Verwendung eines einzigen Kondensators auf die Dauern der einzelnen Impulse der 2. Phase derart eingewirkt wird, dass auf die gesamte elektrische Ladung oder die gesamte Energie dieser 2. Phase eingewirkt wird, damit die Ladung oder die Energie der 2. Phase einen gewünschten Anteil im Verhältnis zur Ladung oder zur Energie der 1. Phase hat, ohne die Dauer dieser 2. Phase zu ändern.

23. Verfahren zur Bildung einer Folge von Impulsen gemäß Anspruch 17 bis 21, **dadurch gekennzeichnet, dass** unter Verwendung eines einzigen Kondensators auf die Dauern der einzelnen Pausen der 2. Phase derart eingewirkt wird, dass die gesamte elektrische Ladung oder die gesamte Energie dieser 2. Phase dosiert wird, damit die Ladung oder die Energie der 2. Phase einen gewünschten Anteil im Verhältnis zur Ladung oder zur Energie der 1. Phase hat, ohne die Dauer dieser 2. Phase zu ändern.

24. Verfahren zur Bildung einer Folge von Impulsen gemäß Anspruch 17 bis 21, **dadurch gekennzeichnet, dass** unter Verwendung eines einzigen Kondensators auf die Dauern der Impulse und der einzelnen Pausen der 2. Phase derart eingewirkt wird, dass die gesamte elektrische Ladung oder die gesamte Energie dieser 2. Phase derart dosiert wird, dass die Ladung oder die Energie der 2. Phase einen gewünschten Anteil im Verhältnis zur Ladung oder zur Energie der 1. Phase hat, ohne die Dauer dieser 2. Phase zu ändern.

## Claims

1. An elementary defibrillation pulse train separated by pauses with a train comprising a succession of post-pause cycles resulting from the chopping of at least one discharge from at least one capacitor in order to obtain a defibrillation action through the heart of a living being, for instance of a patient, **characterized in that** the elementary pulses resulting from the chopping of the discharge from the capacitor(s) and/or the zero energy or potential pauses between these pulses are *variable in duration* according to a *predetermined* modulation law so that, by juxtaposition-junction, cycle after cycle, of each segment of these *pulses* each representing an average value obtained by averaging over a cycle, of at least one determining electrical magnitude for defibrillation, thus having in its planned effect on the heart, a curve with a different predetermined form from that of an exponential discharge, which resulting curve form is physiologically adapted to obtain efficient defibrillation with reduced harmfulness.

2. Defibrillation pulse train according to claim 1, **characterized in that** the elementary pulses forming the pulse train are contained within a two-phase wave envelope.

3. Defibrillation pulse train according to claims 1 or 2, **characterized in that** the modulation law is such that the modulation depth is variable between a low limit and a high limit, the latter making it possible to use the maximum available energy.

4. Defibrillation pulse train according to any one of the previous claims **characterized in that** the curve of a predetermined form is the curve of the cycle by cycle averages obtained in each cycle from each quantity of elementary energy contained in a cycle.

5. Defibrillation pulse train according to the previous claim whereby the elementary energy corresponding to a cycle is identical to that of the previous cycle and that of the next cycle.

6. Defibrillation pulse train according to any one of the previous claims from 1 to 3 **characterized in that** the curve of a predetermined form is the curve of the cycle by cycle averages obtained for each cycle of each value of the current during the cycle.

7. Defibrillation pulse train according to one any of the previous claims from 1 to 3 **characterized in that** the curve of a predetermined form is the curve of the cycle by cycle averages obtained for each cycle of each instantaneous cycle power level.

8. Defibrillation pulse train according to any one of the previous claims **characterized in that** the curve of the cycle by cycle averages of the electrical magnitudes in a cycle has the approximate form of a more or less linear gate pulse parallel to the time axis.

9. Defibrillation pulse train according to any one of the previous claims from 1 to 7, **characterized in that** the curve of the cycle by cycle averages of the electrical magnitude in a cycle has the approximate form of an ascending ramp.

10. Defibrillation pulse train according to any one of the previous claims from 1 to 7, **characterized in that** the curve of the cycle by cycle averages of the electrical magnitude in a cycle has the approximate form of a descending ramp.

11. Defibrillation pulse train according to any one of the previous claims from 1 to 7 **characterized in that** the curve of the cycle by cycle averages of the electrical magnitude in a cycle has the approximate form of a sinusoidal half-wave.

12. Defibrillation pulse train according to any of the previous claims from 1 to 7 **characterized in that** the curve of the cycle by cycle average of the electrical magnitude in a cycle has the approximate form of a sinusoidal half wave.

13. Defibrillation pulse train according to any one of the previous claims from 1 to 7 **characterized in that** the curve of the cycle by cycle average of the electrical magnitude in a cycle has the approximate form of a dome.

14. Defibrillation pulse train according to any one of the previous claims **characterized in that** the curve of the cycle by cycle average of the electrical magnitude obtained in each cycle in the case of the elementary pulses contained in the envelope of a two-phase wave have identical forms.

15. Defibrillation pulse train according to any one of the previous claims from 1 to 13 **characterized in that** the curves of the cycle by cycle averages of the electric magnitude obtained in each cycle in the case of the elementary pulses contained in the envelope of a two-phase wave have alternating inverse or different forms.

16. Defibrillation pulse train according to the previous claim **characterized in that** the curves of the cycle by cycle averages of the electrical magnitude obtained in each cycle in the case of the elementary pulses contained in the envelope of a two-phase wave form an ascending ramp corresponding to the first phase and a gate pulse corresponding to the second phase.

17. Process for the forming of an elementary defibrillation pulse train from a discharge of at least one capacitor **characterized in that** this discharge is chopped into elementary pulses each separated by a pause at zero energy or potential, modulated in duration according to a predetermined modulation law which allows optimal use of the maximum available energy so that the juxtaposition-junction, cycle after cycle, on each of the potential or energy segments each representing an average value obtained each time in a cycle of at least one electrical magnitudes which is determining for defibrillation represents, in its planned effect on the heart, a curve having a different predetermined form from that of an exponential discharge, which resulting curve form is physiologically adapted to obtain efficient defibrillation and reduced harmfulness.

18. Process for the forming of an elementary pulse train according to claim 17 **characterized in that** this determining electrical magnitude is the quantity of energy contained in each cycle.

19. Process for the forming of an elementary pulse train according to the previous claim **characterized in that** the energy corresponding to a cycle is identical to that of the previous cycle and that of the following cycle.

20. Process for the forming of a pulse train according to claim 17 or 18 **characterized in that** the determining electrical magnitude is the current of each cycle.

21. Process for the forming of a pulse train according to claim 17 or 18 **characterized in that** the determining electrical magnitude is the power of each cycle.

22. Process for the forming of a pulse train according to one of claims 17 to 21 **characterized in that**, using a single capacitor, the duration of the individual pulses of the second phase are adjusted in order to dose the total electric charge or the total energy of this second phase so that the charge or the energy of the second phase has the desired proportion with respect to the charge or the energy of the first phase, without changing the duration of this second phase.

23. Process for the forming of a pulse train according to any one of claims 17 to 21 **characterized in that**, using a single capacitor, the durations of the individual pauses of the second phase are adjusted to dose the total electric charge or the total energy of this second phase so that the charge or the energy of the second phase has a desired proportion with respect to the charge or the energy of the first phase, without changing the duration of this second phase.

24. Process for the forming of a pulse train according to any one of the claims 17 to 21 **characterized in that**, using a single capacitor, the durations of the pulses and the individual pauses of the second phase are adjusted to dose the total electric charge or the total energy of this second phase so that the charge or the energy of the second phase has a desired proportion with respect to the charge or the energy of the first phase, without changing the duration of this second phase.
